(19) Europäisches Patentamt
European Patent Office
Office européen des brevets

(11) EP 1 716 888 A1

(12) **EUROPÄISCHE PATENTANMELDUNG**

(43) Veröffentlichungstag:
**02.11.2006 Patentblatt 2006/44**

(21) Anmeldenummer: **05107950.7**

(22) Anmeldetag: **31.08.2005**

(51) Int Cl.:
*A61Q 1/06* (2006.01) *A61K 8/31* (2006.01)
*A61K 8/92* (2006.01) *A61K 8/89* (2006.01)

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **28.04.2005 DE 102005019953**

(71) Anmelder: **Beiersdorf Aktiengesellschaft
D-20253 Hamburg (DE)**

(72) Erfinder:
- **Viehmeyer, Lynn
20251 Hamburg (DE)**
- **Riedel, Cornelia
22889 Tangstedt (DE)**
- **Schwanke, Frank, Dr.
20257 Hamburg (DE)**
- **Lanzendörfer, Ghita, Dr.
90491 Nürnberg (DE)**

(54) **Transferresistente Lippenpflegeprodukte**

(57) Die vorliegende Erfindung betrifft transferresistente Lippenstiftzubereitungen enthaltend
e) ein flüchtiges Öl in einer Konzentration von 40 bis 60 Gew.-%,
f) ein natürliches Wachs in einer Konzentration von 1 bis 10 Gew.-%,
g) ein Silikonharz in einer Menge von 1 bis 10 Gew.-% und
h) ein oder mehre Pigmente.

jeweils bezogen auf das Gesamtgewicht der Formulierung, neben gegebenenfalls weiteren kosmetischen und/oder dermatologischen Wirk-, Hilfs- und Zusatzstoffen und deren Verwendung.

EP 1 716 888 A1

## Beschreibung

Die vorliegende Erfindung betrifft transferresistente Lippenstiftzubereitungen enthaltend

a) ein flüchtiges Öl in einer Konzentration von 40 bis 60 Gew.-%,
b) ein natürliches Wachs in einer Konzentration von 1 bis 10 Gew.-%,
c) ein Silikonharz in einer Menge von 1 bis 10 Gew.-% und
d) ein oder mehre Pigmente.

jeweils bezogen auf das Gesamtgewicht der Formulierung, neben gegebenenfalls weiteren kosmetischen und/oder dermatologischen Wirk-, Hilfs- und Zusatzstoffen und deren Verwendung.

Der Wunsch, schön und attraktiv auszusehen, ist seit Tausenden von Jahren in den Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht. Der Begriff der dekorativen Kosmetik leitet vom lateinischen "decoratio" - das Hervorheben des Schönen — ab. Meist werden dabei mit Hilfe von Farbstoffen einzelne Körperpartien, insbesondere im Gesicht, hervorgehoben und farbliche Uneinheitlichkeiten abgemildert.

Zu den am meisten verwendeten dekorativen Kosmetika gehört der Lippenstift, mehr als 50 % der Frauen in Deutschland verwenden ihn. Meist dient er neben der farblichen Betonung der Lippen auch zur Lippenpflege. Neben dem klassischen Lippenstift werden auch Lip-Gloss und Konturenstifte verwendet. Lip-Gloss verleiht den Lippen einen starken dekorativen Glanz, Konturenstifte dienen der Betonung der Lippenränder.

Die Haut der Lippen besteht aus einer dünnen Hornschicht. Die sich darunter befindliche Lederhaut schiebt gut durchblutete Papillen bis dicht unter die Lippenoberfläche. Daher rührt das natürliche Aussehen der Lippen. Schweißdrüsen sind an den Lippen nicht zu finden. Sie werden hauptsächlich vom Mundspeichel befeuchtet. Auch Talgdrüsen kommen nur vereinzelt vor, so dass die Lippenoberfläche praktisch fettfrei ist. Daher neigen die Lippen, insbesondere an kalten und trockenen Tagen zum Austrocknen und Rissig-werden [W. Umbach (Hrsg): Kosmetik, Entwicklung, Herstellung und Anwendung kosmetischer Mittel (2.Auflage), Thieme-Verlag Stuttgart, 1995].

Lippenpflegeprodukte werden vor allem in Form von Stiften hergestellt, daneben gibt es aber auch Glosse, Creme- und Gelformulierungen.

Die meisten Stiftformulierungen sind wasserfreie Fettmischungen aus festen oder halbfesten Wachsen und flüssigen Ölen, wobei die hochgereinigten Paraffinöle und -wachse die Lippenstiftgrundmasse darstellen. Auch wasserhaltige Zubereitungen sind bekannt. Bei diesen ist der Wassergehalt im Allgemeinen sehr gering. Durch besondere Technologien sind aber auch Wassergehalte bis über 50 % erreichbar in Stiften. Übliche Grundstoffe für stiftförmige Zubereitungen sind beispielsweise flüssige Öle (z. B. Paraffinöle, Ricinusöl, Isopropylmyristat), halbfeste Bestandteile (z. B. Vaseline, Lanolin), feste Bestandteile (z. B. Bienenwachs, Ceresin und Mikrokristalline Wachse bzw. Ozokerit) hochschmelzende Wachse (z. B. Carnaubawachs, Candelillawachs). Lippenstifte des Standes der Technik mit einem Gehalt an Paraffinen und Bienenwachs sind in "Kosmetik, Entwicklung Herstellung und Anwendung kosmetischer Mittel", S. 105, Herausgeber: W. Umbach, Georg Thieme Verlag, Stuttgart - New York, 1988, beschrieben.

Diese wachshaltigen Systeme neigen jedoch zur Übertragung. Um diesem Nachteil des Standes der Technik abzuhelfen, gab es eine Vielzahl von Versuchen, Lippenstiftzubereitungen zu formulieren. In der Regel müssen in diesen Fällen größere Mengen an Filmbildnern eingesetzt werden, um eine transferresistente Zubereitung zu erhalten.

Das europäische Patent EP 979 443 beschreibt Lippenstiftzubereitungen, die zur Steigerung der Transferresistenz hohe Mengen an synthetischen Fettalkoholen enthalten. Solcherlei Zubereitungen weisen eine unkosmetisch feste Konsistenz auf bzw. haben eine unkosmetische Sensorik, das bedeutet sie haben einen öligen Abrieb, sind nicht cremig und hart. Zusätzlich weisen die Zubereitungen einen hohen Tropfpunkt auf und sind somit schwerer herzustellen. Der Schmelzpunkt des in den europäischen Patenten vorzugsweise genannten Fettalkohole Performacol 550 ist 99°C.

Das bedeutet für die Produktion, dass im Fall Fettalkohol Performacol 550 eine Beheizung zum Aufschmelzen mit heißem Wasser nicht mehr ausreicht. Zum Aufschmelzen muss hierzu mit Dampf oder hochsiedenden Wärmeträgerfluiden geheizt werden, was den apparativen Aufwand und den Energiebedarf erhöht.

Die europäischen Patente EP 862 411 und 862 412 beschreiben Lippenfärbezubereitungen, die zur Steigerung der Transferresistenz hohe Mengen an Silikonharzen enthalten. Die Zubereitungen stellen flüssige Lippenfarben dar. Flüssige Lippenfärbezubereitungen sind nicht so einfach zu applizieren wie stiftförmige.

Es war nun die Aufgabe der vorliegenden Erfindung, feste und transferresistente Lippenstiftzubereitungen bereitzustellen, die kosmetisch ansprechend sind, eine einfache Applikation ermöglichen und einen geringen Schmelzpunkt aufweisen.

Überraschend wird die Aufgabe gelöst durch Lippenstiftzubereitungen enthaltend

a) ein flüchtiges Kohlenwasserstoff-Öl in einer Konzentration von 40 bis 60 Gew.-%,

b) ein natürliches Wachs in einer Konzentration von 1 bis 10 Gew.-%,
c) ein Silikonpolymer in einer Konzentration von 1 bis 10 Gew.-% und
d) ein oder mehre Pigmente

jeweils bezogen auf das Gesamtgewicht der Formulierung, neben gegebenenfalls weiteren kosmetischen und/oder dermatologischen Wirk-, Hilfs- und Zusatzstoffen.

Insbesondere vorteilhaft sind transferesistente Lippenstiftzubereitungen enthaltend

a) Isododekan in einer Konzentration von 40 bis 60 Gew.-%,
b) Schellackwachs in einer Konzentration von 1 bis 10 Gew.-%,
c) Silikonharz in einer Menge von 1 bis 10 Gew.-% und
d) ein oder mehre Pigmente

jeweils bezogen auf das Gesamtgewicht der Formulierung, neben gegebenenfalls weiteren kosmetischen und/oder dermatologischen Wirk-, Hilfs- und Zusatzstoffen und deren Verwendung.

Der Schmelzpunkt der Lippenstiftzubereitung lässt sich durch Einsatz von natürlichen Wachsen gegen über den Zubereitungen die reine synthetische Fettalkohole enthalten, signifikant absenken.

Im Fall Schellackwachs muss der Mischung der Einzelkomponenten lediglich auf max. 90°C erhitzt werden, was sehr ökonomisch durch heißes Wasser möglich ist.

Daraus resultieren erhebliche Energieeinsparungen bei der Herstellung, was zu einem entscheidenden Kostenvorteil führt. Auch ist durch die Verkürzung von Aufheiz- und Abkühlungsphasen eine schnellere Produktion möglich.

Lippenstifte aus den erfindungsgemäßen Lippenstiftzubereitungen weisen sensorisch angenehme Eigenschaften auf, da sie cremiger sind und leichter auf den Lippen gleiten.

Optional können die erfindungsgemäßen Zubereitungen Filmbildner enthalten, wodurch eine noch bessere Abriebfestigkeit (Transferresistenz) erreicht wird.

Die neue erfindungsgemäßen Lippenstiftzubereitungen schaffen also den Spagat zwischen Transferresistenz und Tragekomfort. Die erfindungsgemäße Zubereitung weist nach Trocknung kein unangenehm klebriges Gefühl auf und ist nicht spürbar, zudem ist der Film transferresistent.

Erfindungsgemäß vorteilhaft zu verwendende Wachskomponenten können aus der Gruppe der pflanzlichen Wachse, tierischen Wachse, Mineralwachse und fossilen Wachse (petrochemischen Wachsen) gewählt werden.

Erfindungsgemäß günstig sind beispielsweise Candelillawachs, Carnaubawachs, Japanwachs, hydriertes Jojobawachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), fossile Wachse (petrochemische Wachse wie Paraffinwachse und Mikrowachse).

Als Wachse werden erfindungsgemäß ganz besonders bevorzugt eingesetzt solche Substanzen, die einen Schmelzpunkt von über 45°C aufweisen, wie Carnaubawachs, Candelillawachs, Bienenwachs, Schellackwachs, mikrokristalline Wachse und Ozokerite.

Als Wachskomponente der erfindungsgemäßen Zubereitungen ist ganz besonders das gut verfügbare Schellackwachs geeignet.

Zusätzlich zu den natürlichen Wachsen ist es erfindungsgemäß vorteilhaft synthetische Wachse vom Typ Fischer-Tropsch Polymethylen und/oder Fischer-Tropsc Polyethylen, welche einen reinen Kohlenwasserstoff darstellen und nicht funktionalisiert sind, einzusetzen. Bei den aus dem Stand der Technik bekannten synthetischen Wachsen handelt es sich ausnahmslos umfunktionalisierte Wachse, die einen ungünstiges Schmelzverhalten aufweisen. Auch gesättigte Ester unverzweigter Alkankarbonsäuren können neben den natürlichen Wachsen vorteilhaft in den Zubereitungen eingearbeitet sein.

Die erfindungsgemäße Zubereitung enthält weiterhin ein im flüchtigen Öl lösliches oder teilweise lösliches Silikon. Dieses Silikon ist ausgewählt aus der Gruppe der Silikon-Harze. Si-Harze bestehen aus verzweigten Molekülen, welche hauptsächlich aus M-Einheiten und Q-Einheiten aufgebaut sind. Q-Einheiten werden gebildet durch je ein Si-Atom welches mit vier Sauerstoffatomen verbunden ist; es ist keine Methyl-Einheit vorhanden. So wird ein stark verzweigtes Molekülgerüst möglich. Die außerdem in hohem Anteil enthaltenen M-Einheiten enthalten drei Methylgruppen und limitieren den Molekülwachstum.

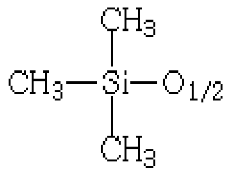

M-Einheit

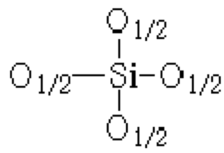

Q-Einheit

Die bevorzugten Si-Harze sind z. B. löslich in Isopropanol, Ethanol, Isopropyl Myristate, kurzkettigen Isoparaffinen, Dimethicone, Cyclomethicone, Phenyl Trimethicone und hinterlassen einen glatten und weichen Film auf der Haut. Sie sind unlöslich in Waser, Glycerin oder Mineralölen.

Besonders bevorzugt sind solche Silikon-Harze, welche eine Molmasse von 1.000 - 20.000 g/mol aufweisen, bei 25°C fest sind, und durch folgende Formel beschrieben werden können:

$$[R_3SiO_{1/2}]_x \cdot [SiO_2]_y$$

wobei das Verhältnis X/Y = 0,4 bis 0,9 beträgt
und R eine Alkylgruppe mit 1-6 C-Atomen ist.

Bevorzugt in Sinne der Erfindung ist das Si-Harz Trimethylsiloxysilicate mit einer mittleren Molekülmasse von 3.000 bis 15.000 g/mol.

Die Ölphase der erfindungsgemäßen Zubereitung enthält ein oder mehrere flüchtige Kohlenwasserstoff-Öle (flüchtige Öle) enthalten, welche vorzugsweise C8-C16 Kohlenwasserstoffe sind. Mischungen möglich. Wobei flüchtig bedeutet bei Raumtemperatur (25°C) flüchtig, mit einem Dampfdruck von 0,01 bis 0,5 kPa bei 20°C. Ein besonders gutes Ergebnis lässt sich erreichen, wenn die flüchtigen Öle einen Dampfdruck von 0,012 bis 0,2 kPa aufweisen.

Bevorzugt sind C8-C16 Isoparaffine, besonders bevorzugt ist Isododecane.

Die kosmetischen und/oder dermatologischen Zubereitungen gemäß der Erfindung können kosmetische Hilfsstoffe enthalten, wie sie üblicherweise in solchen Zubereitungen verwendet werden, z.B. Parfüme, UV-Filter, Verdikkungsmittel, Konservierungsstoffe, oder andere übliche Bestandteile einer kosmetischen oder dermatologischen Formulierung wie Alkohole, Polyole, Polymere, Elektrolyte, organische Lösungsmittel.

Die erfindungsgemäße Lippenstiftzubereitungen enthalten Farbstoffe und/oder Farbpigmente. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch.

Pigmente können organischen und anorganischen Ursprungs sein, wie beispielsweise organische vom Azo-Typ, Indigoide, Triphenylmethan-artige, Anthrachinone, und Xanthin Farbstoffe, die als D&C and FD&C blues, browns, greens, oranges, reds, yellows bekannt sind. Anorganische Pigmente bestehen aus unlöslichen Salzen von zertifizierten Farbstoffen, die als Lakes oder Eisenoxide bezeichnet werden. Beispielsweise können Barium lakes, calcium lakes, aluminum lakes, titandioxide, mica and iron oxides Verwendung finden. Als Al-Salze sind z.B Red 3 Aluminum Lake, Red 21 Aluminum Lake, Red 27 Aluminum Lake, Red 28 Aluminum Lake, Red 33 Aluminum Lake, Yellow 5 Aluminum Lake, Yellow 6 Aluminum Lake, Yellow 10 Aluminum Lake, Orange 5 Aluminum Lake, Blue 1 Aluminum Lake und Kombinationen einsetzbar.

Als Eisenoxide oder-oxidhydrate sind z.B. cosmetic yellow oxide C22-8073 (Sunchemical) cosmetic yellow oxide C33-1700 (Sunchemical), cosmetic brown oxide C33-115 (Sunchemical), cosmetic iron oxide red C33-2199 (Sunchemical), cosmetic russet oxide C33-8075 (Sunchemical), cosmetic iron oxide black C33-5000 (Sunchemical), als Titandioxide z.B. Kronos 1171 (Kronos), C47-051 Cosmetic White (Sunchemical) bekannt und gegebenfalls vorteilhaft.

Ebenso ist es bevorzugt, wenn diese Zubereitungen einen Gehalt an anorganischen Pigmenten gewählt aus der Gruppe der Metalloxide und/oder anderen in Wasser schwerlöslichen oder unlösliche Metallverbindungen, bevorzugt Oxide des Titans ($TiO_2$), Zinks (ZnO), Eisens (z. B. $Fe_2O_3$) Zirkoniums ($ZrO_2$), Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden sowie das Sulfat des Bariums ($BaSO_4$) aufweisen.

Die anorganischen Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natriumhexametaphosphat $(NaPO_3)_6$, Natriummetaphosphat $(NaPO_3)_n$, Siliciumdioxid ($SiO_2$) (auch: Silica, CAS-Nr.: 7631-86-9), oder Eisenoxid ($Fe_2O_3$). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Ferner kann es erfindungsgemäß vorteilhaft sein Perlglanzpigmente einzusetzen.

Dazu zählen natürliche Perlglanzpigmente, wie z. B.

- "Fischsilber" (Guanin/Hypoxanthin-Mischkristalle aus Fischschuppen) und
- "Perlmutt" (vermahlene Muschelschalen),

monokristalline Perlglanzpigmente wie z. B. Bismuthoxychlorid (BiOCl),
Schicht-Substrat Pigmente: z. B. Glimmer / Metalloxid.

Vorteilhaft sind ferner beispielsweise die folgenden Perlglanzpigmentarten auf Basis von Glimmer/Metalloxid:

| *Gruppe* | Belegung / Schichtdicke | Farbe |
|---|---|---|
| Silberweiße Perlglanzpigmente | $TiO_2$: 40 - 60 nm | silber |
| Interferenzpigmente | $TiO_2$: 60-80 nm | gelb |
| | $TiO_2$: 80 -100 nm | rot |
| | $TiO_2$: 100 - 140 nm | blau |
| | $TiO_2$: 120 - 160 nm | grün |
| Farbglanzpigmente | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| Kombinationspigmente | $TiO_2$ / $Fe_2O_3$ | Goldtöne |
| | $TiO_2$ / $Cr_2O_3$ | grün |
| | $TiO_2$ / Berliner Blau | tiefblau |
| | $TiO_2$ / Carmin | rot |

Als besonders bevorzugt sind die Pigmente der Firmen Costenoble (Cloisonne-Typ, Flamenco-Typ, Low Lustre-Typ), Merck (Colorona-Typen, Microna-Typ, Timiron-Typ, Colorona, Ronasphere), Les Colornats Wacker (Covapure, Vert oxyde de Chrome), Cadre (Colorona, Sicopearl), BASF (Sicopearl, Sicovit), Rona (Colorona) bekannt.

Es kann darüber hinaus von Vorteil sein, gänzlich auf ein Substrat wie Glimmer zu verzichten. Besonders

bevorzugt sind Perlglanzpigmente, welche unter der Verwendung von $SiO_2$ hergestellt werden, z. B. solche wie von Merck unter dem Namen Xirona erhältlichen. Solche Pigmente, die auch zusätzlich gonichromatische Effekte haben können, sind z. B. unter dem Handelsnamen Sicopearl Fantastico bei der Firma BASF erhältlich.

Vorteilhaft können Pigmente auf Basis von synthetischem Glimmer (Synthetic Fluorophlogopite) der Firma Sunchemical. Diese sind unter dem Namen SunShine erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihrer Partikelgröße von 40 - 180 μm zusätzlich zu der Farbe einen Glitzereffekt auf.

Weiterhin vorteilhaft können Pigmente der Firma Merck auf Basis von Calcium Aluminium Borosilikat, die mit Titandioxid beschichtet sind, eingesetzt werden. Diese sind unter dem Namen Ronastar erhältlich.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 5,0 bis 15 Gew.-% gewählt, jeweils bezogen auf das Gesamtgewicht der Zubereitungen.

Die Liste der genannten Perlglanzpigmente, Farbstoffe und Farbpigmente, die in den erfindungsgemäßen Lippenprodukten verwendet werden können, soll selbstverständlich nicht limitierend sein.

Erfindungsgemäß ist auch das Verfahren der Einarbeitung von Farbpigmenten in die erfindungsgemäßen Zubereitungen. Dabei werden die Farbpigmente zunächst in einem kosmetischen Öl vordispergiert, bevor diese Dispersion in die Zubereitung eingearbeitet wird. Erfindungsgemäß vorteilhafte Öle zur Herstellung der Dispergierung der Farbstoffe sind vor allem polare Öle, insbesondere Kokosglyceride, Octyldodecanol, Diisostearylfumarat.

Pigmente, insbesondere anorganische Pigmente, können auch als Lichtfilter wirken und so die Lippen vor einer Schädigung durch UV-Strahlung schützen.

Ist es ferner im Sinne der Erfindung, wenn in der lipophilen Phase bei Raumtemperatur flüssige Lipide enthalten sind. Vorteilhaft sind insbesondere die flüssigen Lipide aus der Gruppe der verzweigten und unverzweigten Kohlenwasserstoffe, der Dialkylether, der Gruppe der gesättigten oder ungesättigten, verzweigten oder unverzweigten Alkohole, sowie der Fettsäuretriglyceride, namentlich der Triglyceinester gesättigter und/oder ungesättigter, verzweigter und/oder unverzweigter Alkancarbonsäuren einer Kettenlänge von 8 bis 24, insbesondere 12 - 18 C-Atomen.

Die Fettsäuretriglyceride können beispielsweise vorteilhaft gewählt werden aus der Gruppe der synthetischen, halbsynthetischen und natürlichen Öle, wie z. B. Olivenöl, Sonnenblumenöl, Sojaöl, Erdnussöl, Rapsöl, Mandelöl, Palmöl, Kokosöl, Weizenkeimöl, Traubenkernöl, Distelöl, Nachtkerzenöl, Macadamianussöl und dergleichen mehr.

Erfindungsgemäß vorteilhaft ist beispielsweise der Einsatz von Kokosglyceride (Myritol 331).

Bevorzugt sind weiterhin Lipide gewählt aus der Gruppe der synthetische und natürlichen Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Weitere erfindungsgemäße Öle können gewählt werden aus der Gruppe der Guerbet-Alkohole.

Die Gesamtmenge an Guerbet-Alkoholen in der fertigen Schminkstiftformulierung wird vorteilhaft aus dem Bereich bis 25,0 Gew.-%, bevorzugt 0,5 - 15,0 Gew.-% gewählt, bezogen auf das Gesamtgewicht des Stiftes, wobei vorzugsweise Octyldodecanol eingesetzt wird.

Weiterhin ist es vorteilhaft auch Lipide aus der Gruppe der unpolaren Öle einzusetzen. Das sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und Polyisobutene sowie hydrogenierte Polyisobutene. Unter den Polyolefinen sind Polydecene und Polyisobutene die bevorzugten Substanzen.

Es können beliebige Abmischungen von Öl- und Wachskomponenten vorteilhaft im Sinne der vorliegenden Erfindung eingesetzt werden.

Es ist von Vorteil, das Verhältnis von Wachs- und nicht flüchtigen Lipidkomponenten zueinander ungefähr aus dem Bereich der Gewichtsverhältnisse zwischen 2 : 3 bis 1 : 5, insbesondere 1 : 2 bis 1 : 3, einzustellen.

Weiterhin ist es bevorzugt, wenn die Zubereitungen einen Gehalt an organischen Lichtfiltern enthält. Insbesondere Bisoctyltriazol, 2,4,6-Tris-(biphenyl)-1,3,5-triazin, 2,4,6-Tris-(terphenyl)-1,3,5-triazin Bis-ethylhexyloxyphenolmethoxyphenyltriazin, 4-(tert.-Butyl)-4'-methoxydibenzoylmethan, Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, Phenylen-1,4-bis-(2-benzimidazyl)-3,3'-5,5'-tetrasulfonsäure, Ethylhexyl-2-cyano-3,3-diphenylacrylat (Octocrylen), 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethylhexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methylpyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methylpropyloxy)-2-hydroxy]-phenyl}-6-(4-methoxyphenyl)-1,3,5-triazin, 1,4-di(2-oxo-10-Sulfo-3-bornylidenmethyl)-Benzol, Hydroxybenzophenonderivate, 2-(4'-Diethylamino-2'-hydoxybenzoyl)-benzoesäurehexylester, Homomenthylsalicylat, Bis-Resorcinyltriazinderivate, 2-Ethylhexyl-2-cyano-3,3-diphenylacrylat, 2-Ethylhexyl-2-hydroxybenzoat, 2-(4'-(Diethylamino)-2'-hydoxybenzoyl)-benzoesäurehexylester und Ester der Zimtsäure, bevorzugt 4-Methoxyzimtsäure(2-ethylhexyl)ester und/oder 4-Methoxyzimtsäureisopentylester haben sich als vorteilhaft herausgestellt.

Bei all diesem ist es bevorzugt, wenn der Gehalt an UV-Filtersubstanzen 0,1 Gew.-% bis 20 Gew.-%, besonders bevorzugt 0,5 bis 15 Gew.-%, ganz besonders bevorzugt 1,0 bis 12,0 Gew.-%, jeweils bezogen auf das Gesamtgewicht der Zubereitungen, beträgt.

Erfindungsgemäß können auch lipophilen Wirkstoffe sehr vorteilhaft in die Lippenprodukte eingearbeitet werden. Insbesondere geeignet sind:

Acetylsalicylsäure, Atropin, Azulen, Hydrocortison und dessen Derivaten, z. B. Hydrocortison-17-valerat, Vitamine, z. B. Ascorbinsäure und deren Derivate, Vitamine der B- und D-Reihe, sehr günstig das Vitamin $B_1$, das Vitamin $B_{12}$ das Vitamin $D_1$, Vitamin E und Vitamin E Acetate aber auch Bisabolol, ungesättigte Fettsäuren, namentlich die essentiellen Fettsäuren (oft auch Vitamin F genannt), insbesondere die $\gamma$-Linolensäure, Ölsäure, Eicosapentaensäure, Docosahexaensäure und deren Derivate, Chloramphenicol, Coffein, Prostaglandine, Thymol, Campher, Extrakte oder andere Produkte pflanzlicher und tierischer Herkunft, z. B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl®, Eucerit® und Neocerit®.

Es ist dem Fachmanne natürlich bekannt, dass anspruchsvolle kosmetische Zubereitungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Weiterhin kann es von Vorteil sein, durch den Zusatz von so genannten Füllstoffen die sensorischen Eigenschaften des Stiftes zu modifizieren.

Füllstoffe im Sinne der vorliegenden Erfindung sind partikuläre Substanzen, die in der Regel keinen Farbeffekt in der kosmetischen Formulierung erzeugen, in der sie eingesetzt werden. Ferner haben erfindungsgemäße Füllstoffe üblicherweise einen niedrigen Brechungsindex und daraus resultierend keine oder eine nur sehr geringe Deckkraft.

Der Stand der Technik kennt eine Reihe von Füllstoffen, welche z. B. als Trägermaterialien bei der Formulierung von Pudern oder als Viskositäts- und Sensorik-Modulatoren in Emulsionen oder wasserfreien Formulierungen dienen. Darüber hinaus beeinflusst der Einsatz von Füllstoffen im allgemeinen auch die Verteilbarkeit üblicher Formulierungen auf der Haut sowie die Gleichmäßigkeit eines möglichen Farbeffektes.

Geeignete Füllstoffe im Sinne der Erfindung sind Bornitrid, Nylon-12, Polymethylsilsesquioxane, Polymethylmethycrylate, Polyethylene, Polytetrafluorethylene, Lauroyl Lysine , Silica, Silica Dimethyl Silylate, Glimmer/Mica, Talkum, Kaolin sowie Stärke bzw. Stärkederivate. Die Füllstoffe können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll.

Derartige Zubereitungen werden bevorzugt verwendet zur Herstellung von geschmacksneutralen Lippenpflegezubereitungen und/oder Lippendekorationszubereitungen.

Um dem Lippenstift einen Geschmack zu geben bzw. den Eigengeschmack der Inhaltsstoffe zu überdecken, lassen sich viele aus der Lebensmittelindustrie bekannte Aromen einarbeiten.

Die erfindungsgemäßen kosmetischen und/oder dermatologische Zubereitungen werden vorteilhafterweise in einer Lippenstifthülse angeboten. Dafür wird die Lippenstiftmasse in eine Form gegossen und der Gießling nach dem Abkühlen in das Pfännchen der Hülse platziert. Alternativ kann der Stift auch direkt in spezielle Packmittel gegossen werden, so genanntes *direct filling.* Unabdingbar für die Stabilität des Stiftes in der Hülse ist, dass diese Hülse aus diffusionsdichtem Material besteht oder mit diffusionsdichten Material beschicht ist, um ein Abdampfen des flüchtigen Öls bei der Lagerung zu verhindern. Des weiteren muss der Stift beim Nichtgebrauch dicht verschließbar sein.

Die nachfolgenden Beispiele sollen die Erfindung erläutern, ohne sie einzuschränken.

Alle Mengenangaben, Prozentangaben oder Teile beziehen sich, soweit nicht anders angegeben, auf das Gewicht, insbesondere auf das Gesamtgewicht der Zubereitungen oder der jeweiligen Mischungen.

Transferresistente Lippenstiftzubreitungen:

| | 1 | 2 | 3 | 4 |
|---|---|---|---|---|
| Synthetic Wax (Fischer Tropsch, only C and H) | 11 | 10 | 12 | 15 |
| Shellac Wax | 2,5 | 4 | 1,5 | 2 |
| Hydrated Jojobawax | | | 0,5 | |
| Isododecane | 50 | 40 | 50 | 55 |
| Pigments | 10 | 10 | 10 | 10 |
| Trimethylsiloxysilicate | 10 | 8 | 5 | 12 |
| Sucrose Acetate Ester | | 2 | | |
| PVP/Hexadecene Copolymer | 2 | | | 3 |
| PVP/Eicosene Copolymer | | | 1 | |
| Propylparaben | 0,1 | 0,1 | 0,1 | 0,1 |
| BHT | 0,03 | 0,03 | 0,03 | 0,03 |
| Polyisobutene | add to 100 | | | |
| Hydrogenated Polydecene | | add to 100 | | |
| Hydrogenated Polyisobutene | | | | add to 100 |
| Jojoba oil | | | add to 100 | |
| | 5 | 6 | 7 | |
| Synthetic Wax (Fischer Tropsch, only C and H) | 10 | 10 | 12 | |
| Shellac Wax | 5 | | 3 | |
| Hydrated Jojobawax | | 4 | | |
| Isododecane | 50 | 45 | 60 | |
| Pigments | 10 | 10 | 10 | |
| Trimethylsiloxysilicate | 6 | 4 | 5 | |
| Sucrose Acetate Ester | 2 | | | |
| PVP/Eicosene Copolymer | | | 1 | |
| Propylparaben | 0,1 | 0,1 | 0,1 | |
| BHT | 0,03 | 0,03 | 0,03 | |
| Polyisobutene | add to 100 | | | |
| Hydrogenated Polydecene | | | add to 100 | |
| Hydrogenated Polyisobutene | | add to 100 | | |

**Patentansprüche**

**1.** Kosmetische und/oder dermatologische Lippenstiftzubereitungen enthaltend

a) ein flüchtiges Kohlenwasserstoff-Öl in einer Konzentration von 40 bis 60 Gew.-%,

b) ein natürliches Wachs in einer Konzentration von 1 bis 10 Gew.-%,
c) ein Silikonpolymer in einer Konzentration von 1 bis 10 Gew.-% und
d) ein oder mehrere Farbpigmente in einer Konzentration von 0,1 bis 20 Gew.-%, jeweils bezogen auf das Gesamtgewicht der Formulierung, neben gegebenenfalls weiteren kosmetischen und/oder dermatologischen Wirk-, Hilfs- und Zusatzstoffen.

**2.** Kosmetische und/oder dermatologische Lippenstiftzubereitungen nach Anspruch 1, **dadurch gekennzeichnet, dass** das natürliche Wachse gewählt wird aus der Gruppe Candelillawachs, Carnaubawachs, Japanwachs, hydriertes Jojobawachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

**3.** Kosmetische und/oder dermatologische Lippenstiftzubereitungen nach mindestens einem der vorhergehenden Ansprüche, **dadurch gekennzeichnet, dass** das oder die flüchtigen Öle einen Dampfdruck von 0,01 bis 0,5 kPa bei 20°C, insbesondere einen Dampfdruck von 0,012 bis 0,2 kPa bei 20 °C aufweisen.

**4.** Kosmetische und/oder dermatologische Lippenstiftzubereitungen nach mindestens einem der vorhergehenden Ansprüche, **dadurch gekennzeichnet, dass** das Silikonpolymer bei 25°C fest ist, die Formel

$$[R_3SiO_{1/2}]_x\ [SiO_2]_y$$

hat, wobei das Verhältnis X/Y = 0,4 bis 0,9 beträgt und R eine Alkylgruppe mit 1-6 C-Atomen ist und die mittlere Molmasse 1.000 — 20.000 g/mol beträgt.

**5.** Kosmetische und/oder dermatologische Lippenstiftzubereitungen nach mindestens einem der vorhergehenden Ansprüche, **dadurch gekennzeichnet, dass** das Silikonpolymer ein Trimethylsiloxysilicate mit einer mittleren Molekülmasse von 3.000 bis 15.000 g/mol ist.

**6.** Kosmetische und/oder dermatologische Lippenstiftzubereitungen enthaltend

a) Isododekan in einer Konzentration von 40 bis 60 Gew.-%,
b) Schellackwachs in einer Konzentration von 1 bis 10 Gew.-%,
c) Trimethylsiloxysilicate in einer Menge von 1 bis 10 Gew.-% und
d) ein oder mehrere Farbpigmente in einer Konzentration von 0,1 bis 20 Gew.-%, jeweils bezogen auf das Gesamtgewicht der Formulierung, neben gegebenenfalls weiteren kosmetischen und/oder dermatologischen Wirk-, Hilfs- und Zusatzstoffen.

**7.** Verwendung der kosmetischen und/oder dermatologischen Zubereitung nach einem der vorhergehenden Ansprüche als transferresistenter Lippenstift oder ein transferresistentes Lippenstiftprodukt.

Europäisches Patentamt

# EUROPÄISCHER RECHERCHENBERICHT

Nummer der Anmeldung
EP 05 10 7950

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 386 600 A (L'OREAL) 4. Februar 2004 (2004-02-04) * Beispiel 9 * | 1-4 | INV. A61Q1/06 A61K8/31 A61K8/92 A61K8/89 |
| X | US 2004/161395 A1 (PATIL ANJALI ABHIMANYU ET AL) 19. August 2004 (2004-08-19) * Absätze [0008], [0267]; Ansprüche; Beispiel 1 * | 1-7 | |
| X | US 6 060 072 A (KONIK ET AL) 9. Mai 2000 (2000-05-09) * Anspruch 1; Beispiel 1 * | 1-7 | |
| A | US 6 406 683 B1 (DRECHSLER LEE ELLEN ET AL) 18. Juni 2002 (2002-06-18) * Beispiele * | 1-7 | |
| A | US 2004/258642 A1 (CALELLO JOSEPH FRANK ET AL) 23. Dezember 2004 (2004-12-23) * Beispiele * | 1-7 | |
| | | | RECHERCHIERTE SACHGEBIETE (IPC) |
| | | | A61Q A61K |

Der vorliegende Recherchenbericht wurde für alle Patentansprüche erstellt

2

| Recherchenort | Abschlußdatum der Recherche | Prüfer |
|---|---|---|
| München | 24. Juli 2006 | Miller, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 05 10 7950

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-07-2006

| Im Recherchenbericht angeführtes Patentdokument | Datum der Veröffentlichung | Mitglied(er) der Patentfamilie | Datum der Veröffentlichung |
|---|---|---|---|
| EP 1386600 A | 04-02-2004 | JP 2004131475 A | 30-04-2004 |
| US 2004161395 A1 | 19-08-2004 | WO 2004073626 A2 | 02-09-2004 |
| US 6060072 A | 09-05-2000 | AT 298558 T | 15-07-2005 |
| | | AU 746186 B2 | 18-04-2002 |
| | | AU 1126199 A | 24-05-1999 |
| | | CA 2276031 A1 | 14-05-1999 |
| | | DE 69830715 D1 | 04-08-2005 |
| | | EP 0969809 A1 | 12-01-2000 |
| | | JP 2001503071 T | 06-03-2001 |
| | | WO 9922711 A1 | 14-05-1999 |
| | | US 5959009 A | 28-09-1999 |
| US 6406683 B1 | 18-06-2002 | KEINE | |
| US 2004258642 A1 | 23-12-2004 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 979443 A
- EP 862411 A
- EP 862412 A

### In der Beschreibung aufgeführte Nicht-Patentliteratur

- **W. UMBACH.** Kosmetik, Entwicklung Herstellung und Anwendung kosmetischer Mittel. Georg Thieme Verlag, 1988, 105